# EUROPEAN PATENT APPLICATION

(11) **EP 1 354 953 A1**
(43) Date of publication of application: **22.10.2003**
(21) Application number: 02015499.3
(22) Date of filing: 12.07.2002
(51) Int. Cl.: C12N 15/12, C12N 15/62, A61K 47/48, C07K 5/103, C07K 19/00, C07K 14/47, A61K 38/17

(54) **Smac-peptides as therapeutics against cancer and autoimmune diseases**

(30) Priority: 17.04.2002 EP 02008199
(71) Applicant: Deutsches Krebsforschungszentrum Stiftung des öffentlichen Rechts, 69120 Heidelberg (DE)
(72) Inventor: Debatin, Klaus, Michael, 89075 Ulm (DE); Fulda, Simone, 89075 Ulm (DE)
(74) Representative: Isenbruck, Günter, Dr.

(57) **Abstract**

The invention is directed to the use of Smac to sensitize different tumors and self-reactive immune cells to various pro-apoptotic stimuli, in that the cells subsequently undergo apoptosis. Therefore, Smac can be used as a compound for the manufacture of a medicament for the treatment of cancer and autoimmune diseases. Sensitization of the cells is achieved either by applying a cell-permeable form of Smac combined with known anticancer agents or by overexpression of the protein. It is an object of the invention to provide a new method in cancer and autoimmune disease therapy by using Smac agonists for apoptosis regulation. Thus, Smac agonists represent novel promising cancer and autoimmune disease therapeutics to potentiate the efficacy of cytotoxic therapies even in resistant tumors and immune cells.

## Description

The present invention relates to the use of the so-called Smac protein and derivatives thereof to cause apoptosis in cancer cells and self-reactive cells of the immune system.

Cancer constitutes the fourth leading cause of death in Western countries. As the average age in the Western population steadily rises, so do cancer-related deaths indicating that cancer will be one of the most common causes of death in the 21^{st} century. The aggressive cancer cell phenotype is the result of a variety of genetic and epigenetic alterations leading to deregulation of intracellular signaling pathways. Cancer cells commonly fail to undergo so-called "programmed cell death" or "apoptosis", a signaling process that plays a key role in preventing cell tissues from abnormal growth. Thus, apoptosis defects appear to be a major problem in cancer therapy as they confer resistance to many tumors against current treatment protocols, leading to tumor progression.

In addition to apoptosis defects found in tumors, defects in the ability to eliminate self-reactive cells of the immune system due to apoptosis resistance are considered to play a key role in the pathogenesis of autoimmune diseases. Autoimmune diseases are characterized in that the cells of the immune system produce antibodies against own organs and molecules or directly attack tissues resulting in the destruction of the latter. A failure of those self-reactive cells to undergo apoptosis leads to the manifestation of the disease. Defects in apoptosis regulation have been identified in autoimmune diseases such as Lupus erythematodes disseminatus or rheumatoid arthritis.

Apoptosis pathways involve diverse groups of molecules. One set of mediators implicated in apoptosis are so-called caspases, cysteine proteases that cleave their substrates specifically at aspartate residues. Caspases convey the apoptotic signal in a proteolytic cascade, with caspases cleaving and activating other caspases which subsequently degrade other cellular targets eventually resulting in cellular breakdown. If one or more steps in this cascade is inhibited in tumor cells, these cells fail to accomplish apoptosis and, thus, continue to grow. Caspase activation itself can be triggered by external stimuli affecting certain cell surface receptors, known to the person skilled in the art as so-called death receptors, or by intracellular stress response via the mitochondria leading to the release of mitochondrial proteins. Known death receptors mediating apoptosis include members of the tumor necrosis factor (TNF) receptor superfamily such as CD95 (APO-1/Fas) or TRAIL (TNF-related apoptosis inducing ligand) receptors 1 and 2. Stimulation of death receptors with apoptosis-inducing substances leads, among others, to the activation of caspase-8, which in turn activates other caspases and members of another group of apoptosis mediators. This group is called the Bcl-2 family and is thought to regulate the release of the mitochondrial proteins and, thus, link both pathways together, in order to regulate the downstream acting proteolytic caspase cascade.

A failure in activating the caspase cascade is caused by the action of so-called Inhibitors of Apoptosis Proteins (IAPs). IAPs bind to early active caspases, thereby preventing the ongoing of the apoptosis process. They are expressed at high levels in many tumors and, by inhibition of caspases, contribute to the resistance of cancers against apoptosis induction.

A major role in activating the caspase cascade is ascribed to a mammalian protein called Smac in humans (or DIABLO in mice). As disclosed, among others, by Du et al. (Cell 102, 2000, 33-42), Smac is a mitochondrial protein of 239 aminoacids possessing a molecular weight of approximately 25000 Dalton (GenBank accession number AAF87716). In the course of an apoptotic response e.g. upon stimulating CD95- or TRAIL death receptors, Smac is released from mitochondria along with other proteins, e.g. cytochrome c. It has been demonstrated earlier that Smac, once released into the cytosol, can bind to IAPs (Du et al. 2000; Verhagen et al. 2000; Srinivasula et al. 2001), particularly to the so-called X-linked IAP (XIAP), the most potent inhibitor of caspases. Binding of Smac to XIAP promotes the proteolytic activation of caspases resulting in apoptosis.

Similar to cancer cells in which activation of caspases is inhibited by IAP-dependent mechanisms, failure to eliminate autoreactive T-cells may be due to a blockade in apoptosis signalling. For physiological elimination of activated lymphocytes death receptor systems such as CD95 play a key role. Increased expression of IAPs or members of the Bcl-2 family in activated T-cells prevents the release of Smac from mitochondria and inhibits the function of the latter.

From the foregoing, it becomes evident that impaired release of Smac and other proteins from mitochondria into the cytosol can cause resistance of tumor cells and cells of the immune system to apoptosis. Overexpression of Smac by transfecting the cells with an expression plasmid carrying the Smac gene is one way to overcome the IAP-caused inhibition of caspases, resulting in an enhanced apoptosis rate. This approach was followed by different research groups, which have found that various types of cancer can thus be treated, e.g. melanoma, breast carcinoma or prostate cancer. However, previous studies do not mention or give any hint to treat neuroblastoma or glioblastoma by overexpressing Smac or related proteins.

A direct delivery of proteins into cells is often limited by the poor permeability of the cell membrane. Recently, Carson et al. (Cancer Research 62 (2002) 18-23) have used purified Smac which was microinjected alone or together with cytochrome c into the cytosol of prostate cancer cells which were initially resistant to apoptosis. However, various problems can be encountered when using microinjection for the delivery of biologically active compounds into cells. Problems include low transfer efficiency or complex manipulation, which would preclude their routine use *in vivo*.

The object of the present invention is to provide a form of Smac that is rapidly internalized into tumor cells and cells of the immune system, e.g. T-cells, by cellular uptake.

This object is attained by a Smac protein / carrier entity comprising
(i) a Smac protein, as disclosed by the GenBank accession number AAF87716, or a derivative or fragment thereof,
(ii) a carrier
and wherein the Smac protein, fragment or derivative thereof and the carrier are linked together enabling the penetration of the Smac/carrier entity through the cell membrane into the cell.

Said entity will be referred to as Smac/carrier entity hereinafter.

A further object of the invention is the therapy of cancers and autoimmune diseases which, until now, could not be treated using Smac proteins.

In the context of the present invention, the term derivative or fragment of the Smac protein refers to peptides in which one or more aminoacids of the sequence of 239 aminoacids, as disclosed in GenBank number AAF87716, can be substituted by one or more aminoacids different from the original one(s), or peptides the aminoacid sequence of which is either extended, shortened, or both, on either the aminoterminal, or the carboxyterminal or both ends with respect to the original Smac proteins, provided that the function of the Smac protein remains unaffected.

In a further embodiment, the present invention includes preferably a peptide comprising aminoacids 56 to 70 of Smac. An even more preferred peptide comprises aminoacids 56 to 62 of Smac. Hereinafter, the latter will be referred to as Smac peptide.

Most preferably, said derivatives or fragments contain the 4 aminoterminal aminoacids 56 to 59 of Smac. This region mediates the interaction of the Smac protein with IAPs.

The carrier, which is preferably a protein, a fragment or derivative thereof, serves as a vehicle the attachment of which to the Smac protein, fragment or derivative thereof enables the penetration of the Smac/carrier entity through the cell membrane into the cell. Appropriate carriers, in particular proteins, are known to the person skilled in the art and include TAT, influenza virus hemagglutinin, the VP22 protein from herpes simplex virus, Antennapedia, fibroblast growth factor, Galparan (transportan), poly-arginine, Pep-1. Other carriers known to a person skilled in the art which do not belong to proteins, but mediate the internalization of molecules into cells include lipids and cationic lipids.

When a protein is used as a carrier, the term derivative or fragment of a protein refers to peptides in which one or more aminoacids can be substituted by other aminoacids different from the original one(s), or peptides the aminoacid sequence of which is either extended, shortened, or both, on either the aminoterminal, or the carboxyterminal or both ends, with respect to the original one(s), provided that the function as a carrier for the cellular uptake of Smac remains unaffected. The above definition relates to TAT, influenza virus hemagglutinin, the VP22 protein from herpes simplex virus, Antennapedia, fibroblast growth factor, Galparan (transportan), poly-arginine and Pep-1.

The Smac protein, fragment or derivative thereof is linked to the carrier. This can occur by any chemical interaction known to the person skilled in the art, like coordinative bonds, chemical adsorption, dipole-dipole interaction or the like. Preferably, the carrier is linked to the Smac protein by a chemical bond, in particular a covalent bond, in case the carrier is a protein. This bond must be such that it remains unaffected before and while penetrating the cell membrane and, if necessary for the interaction of the Smac protein with IAPs, can be cleaved. In general, the Smac/carrier entity can interact with IAPs to the necessary extent, a cleavage being not necessary.

In a preferred embodiment of the present invention, the carrier is TAT or a derivative or a fragment thereof. TAT is the human immunodeficiency virus-1 (HIV-1) trans-activating protein consisting of 86 aminoacids. More preferably, the fragment or derivative of TAT comprises the aminoacids 37 to 72 of TAT, as disclosed in GenBank accession number CAA45921. It is even more preferred to use, as a carrier, the so-called protein transduction domain of TAT (PTD) which comprises a region on the protein extending from aminoacid residues 47 to 57, according to the disclosed sequence. In this preferred embodiment of the invention, PTD is linked to Smac, or a fragment or derivative thereof.

The Smac/carrier entity as disclosed in the present invention can be used as a pharmaceutical, optionally in combination with at least one active compound. This is a further embodiment of the present invention. The term "active compound" refers to a compound other than Smac, a fragment or derivative thereof, which is able to induce apoptosis or which inhibits cell proliferation.

Active compounds which are able to induce apoptosis are known to the person skilled in the art. One class of active compounds are chemical compounds having a cytostatic or antineoplastic effect ("cytostatic compound"). Cytostatic compounds included in the present invention comprise, but are not restricted to (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphteria toxin; (vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as PS-341, phenylbutyrate, ET-18-OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists.

Other cytostatic compounds, which are included in the present invention, include plant-derived cytostatics (from Viscum and derivatives); alcaloids such as vindesine; podophyllotoxins such as vinorelbin; alkylants such as nimustrine, carmustrine, lomustine, estramustrine, melphalam, ifosfamide, trofosfamide, bendamustine, dacarbazine, busulfane, procarbazine, treosulfane, tremozolamide, thiotepa; cytotoxic antibiotics such as aclarubicine, daunorubicine, epirubicine, idarubicine, mitomycine, dactinomycine; antimetabolites like folic acid analogs such as methotrexate, purine analogs such as cladribin, mercaptopurin, tioguanine and pyrimidine analogs such as cytarabine, fluorouracil, docetaxel; platinum compounds such as thioplatin, carboplatin, oxaliplatin; amsacrine, irinotecane, interferon-α, tretinoine, hydroxycarbamide, miltefosine, pentostatine, aldesleukine; antineoplastic compounds derived from organs, e.g. monoclonal antibodies such as trastuzumab, rituximab, or derived from enyzmes such as pegaspargase; endocrine effecting antineoplastic compounds belonging to hormones, e.g. estrogens such as polyestradiol, fosfestriol, ethinylestradiol, gestagens such as medroxyprogesterone, gestonoroncaproat, megestrol, norethisterone, lynestrenol, hypothalamus hormones such as triptoreline, leuproreline, busereline, gosereline, other hormones such as testolactone, testosterone; endocrine effecting antineoplastic compounds belonging to hormone antagonists, e.g. antiestrogens such as toremifen; antiandrogens such as flutamide, bicalutamide, cyproterane; endocrine effecting antineoplastic compounds belonging to enzyme inhibitors such as anastrol, exemestane, letrozol, formestane, aminoglutethimide, all of which can be occasionally administered together with so-called protectives such as calciumfolinat, amifostin, lenograstin, molgromostin, filgrastin, mesna or so-called additives such as retinolpalmitate, thymus D9, amilomer.

Another class of active compounds which can be used in the present invention are those which are able to induce apoptosis by binding to death receptors ("death receptor ligands"). They include tumor necrosis factor α (TNF-α), tumor necrosis factor β (TNF-β, lymphotoxin-α), LT-β (lymphotoxin-β), TRAIL (Apo2L), CD95 (Fas, APO-1) ligand, TRAMP (DR3, Apo-3) ligand, DR4 ligand, DR6 ligand as well as fragments and derivatives of any of said ligands. Preferably, the death receptor ligand is selected from the group consisting of TNF-α, a fragment or derivative thereof, and TRAIL, a fragment and derivative thereof.

Other active compounds include agonistic antibodies to death receptors such as anti-CD95 antibody, anti-TRAIL-R1 (DR4) antibody, anti-TRAIL-R2 (DR5) antibody, anti-DR6 antibody, anti TNF-R1 antibody and anti-TRAMP (DR3) antibody as well as fragments and derivatives of any of said antibodies. Preferably, the agonistic antibodies are selected from the group consisting of anti-TRAIL-R1 antibody, anti-TRAIL-R2 antibody, anti TNF-R1 antibody and fragments and derivatives of any of said antibodies.

The preferred Smac/carrier entity of the present invention is the Smac peptide linked to PTD, and will be referred to as Smac peptide/PTD hereafter.

In the present invention, the cytostatic compound used in combination with the Smac/carrier entity is preferably selected from the group consisting of doxorubicin, cisplatin and etoposide (VP-16). Further preferred active compounds of the present invention used in combination with the Smac/carrier entity are selected from the group of death receptor agonists consisting of TRAIL, anti-CD95 antibody and derivatives and fragments of any of said agonists.

The Smac/carrier entity can be administered alone or in combination with one or more active compounds. The latter can be administered before, after or simultaneously with the administration of the Smac/carrier entity. The dose of either the Smac/carrier entity or the active compound as well as the duration and the temperature of incubation can be variable and depends on the target that is to be treated.

A further object of the present invention are pharmaceutical preparations which comprise an effective dose of at least one Smac/carrier entity and/or at least one active compound and a pharmaceutically acceptable carrier, i.e. one or more pharmaceutically acceptable carrier substances and/or additives.

The pharmaceutical according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The preparation of the pharmaceutical compositions can be carried out in a manner known per se. To this end, the Smac/carrier entity and/or the active compound, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiological sodium chloride solution, alcohols such as ethanol, glycerol, polyols, sucrose, invert sugar, glucose, mannitol, vegetable oils, etc. It is also possible to lyophilize the Smac/carrier entity and/or the active compound and to use the resulting lyophilisates, for example, for preparing preparations for injection or infusion. Suitable carriers for microcapsules, implants or rods are, for example, copolymers of glycolic acid and lactic acid.

The pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the Smac/carrier entity, in combination with one or more active compounds to be administered, depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to the Smac/carrier entity.

The Smac/carrier entities according to the present invention, respectively the medicaments containing the latter, can be used for the treatment of all cancer types which are resistant to apoptosis due to the expression of IAPs. Examples of such cancer types comprise neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma.

Examples of cancer types where the use of the Smac/carrier entities according to the present invention, respectively the medicaments containing the latter, is particularly advantageous include neuroblastoma, glioblastoma, breast carcinoma, melanoma, prostate carcinoma, pancreatic carcinoma, hepatocellular carcinoma, colon carcinoma, small cell and non-small cell lung carcinoma.

The Smac/carrier entities according to the present invention, respectively the medicaments containing the latter, can furthermore be used for the treatment of all autoimmune diseases which are resistant to apoptosis due to the expression of IAPs or members of the Bcl-2 family. Examples of such autoimmune diseases are collagen diseases such as rheumatoid arthritis, Lupus erythematodes disseminatus, Sharp syndrome, CREST syndrome (calcinosis, Raynaud syndrome, esophageal dysmotility, teleangiectasia), dermatomyositis, vasculitis (Morbus Wegener) and Sjögren syndrome, renal diseases such as Goodpasture syndrome, rapidly-progressing glomerulonephritis and membrane-proliferative glomerulonephritis type II, endocrine diseases such as type-I diabetes, autoimmune polyendocrinopathy-candidiasis-ectodermal dystrophy (APECED), autoimmune parathyreoidism, pernicious anemia, gonad insufficiency, idiopathic Morbus Addison, hyperthyreosis, Hashimoto thyreoiditis and primary myxedemia, skin diseases such as Pemphigus vulgaris, bullous pemphigoid, Herpes gestationis, Epidermolysis bullosa and Erythema multiforme major, liver diseases such as primary biliary cirrhosis, autoimmune cholangitis, autoimmune hepatitis type-1, autoimmune hepatitis type-2, primary sclerosing cholangitis, neuronal diseases such as multiple sclerosis, Myastenia gravis, myasthenic Lambert-Eaton syndrome, acquired neuromyotony, Guillain-Barre syndrome (Müller-Fischer syndrome), Stiff-man syndrome, cerebellar degeneration, ataxia, opsoklonus, sensoric neuropathy and achalasia, blood diseases such as autoimmune hemolytic anemia, idiopathic thrombocytopenic purpura (Morbus Werlhof), infectious diseases with associated autoimmune reactions such as AIDS, Malaria and Chagas disease.

In a further embodiment of the present invention neuroblastoma and glioblastoma cells or self-reactive cells of the immune system are treated by administering an active compound in combination with the overexpression of Smac in the cells. The latter is achieved by methods known to persons skilled in the art, preferably by transfecting the cells with an expression plasmid carrying the full length Smac gene, as disclosed in GenBank number AF262240, or a derivative or a fragment thereof.

Active compounds which can be used in the above treatment include cytostatic compounds from the group of antimetabolites, such as cytarabine, fludarabine, 5-fluoro-2'-deoxyuiridine, gemcitabine, hydroxyurea or methotrexate; DNA-fragmenting agents, such as bleomycin, DNA-crosslinking agents, such as chlorambucil, cisplatin, cyclophosphamide or nitrogen mustard; intercalating agents such as adriamycin (doxorubicin) or mitoxantrone; protein synthesis inhibitors, such as L-asparaginase, cycloheximide, puromycin or diphteria toxin; topoisomerase I poisons, such as camptothecin or topotecan; topoisomerase II poisons, such as etoposide (VP-16) or teniposide; microtubule-directed agents, such as colcemid, colchicine, paclitaxel, vinblastine or vincristine; kinase inhibitors such as flavopiridol, staurosporin, STI571 (CPG 57148B) or UCN-01 (7-hydroxystaurosporine); miscellaneous investigational agents such as PS-341, phenylbutyrate, ET-18-OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; hormones such as glucocorticoids or fenretinide; hormone antagonists, such as tamoxifen, finasteride or LHRH antagonists; plant-derived cytostatics (from Viscum and derivatives); alcaloids such as vindesine; podophyllotoxins such as vinorelbin; alkylants such as nimustrine, carmustrine, lomustine, estramustrine, melphalam, ifosfamide, trofosfamide, bendamustine, dacarbazine, busulfane, procarbazine, treosulfane, tremozolamide, thiotepa; cytotoxic antibiotics such as aclarubicine, daunorubicine, epirubicine, idarubicine, mitomycine, dactinomycine; antimetabolites like folic acid analogs such as methotrexate, purine analogs such as cladribin, mercaptopurin, tioguanine and pyrimidine analogs such as cytarabine, fluorouracil, docetaxel; other antineoplastic, platinum compounds such as thioplatin, carboplatin, oxaliplatin; amsacrine, irinotecane, interferon-α, tretinoine, hydroxycarbamide, miltefosine, pentostatine, aldesleukine; antineoplastic compounds derived from organs, e.g. monoclonal antibodies such as trastuzumab, rituximab, or derived from enyzmes such as pegaspargase; endocrine effecting antineoplastic compounds belonging to hormones, e.g. estrogens such as polyestradiol, fosfestriol, ethinylestradiol, gestagens such as medroxyprogesterone, gestonoroncaproat, megestrol, norethisterone, lynestrenol, hypothalamus hormones such as triptoreline, leuproreline, busereline, gosereline, other hormones such as testolactone, testosterone; endocrine effecting antineoplastic compounds belonging to hormone antagonists, e.g. antiestrogens such as toremifen; antiandrogens such as flutamide, bicalutamide, cyproterane; endocrine effecting antineoplastic compounds belonging to enzyme inhibitors such as anastrol, exemestane, letrozol, formestane, aminoglutethimide, all of which can be occasionally administered together with so-called protectives such as calciumfolinat, amifostin, lenograstin, molgromostin, filgrastin, mesna or so-called additives such as retinolpalmitate, thymus D9, amilomer.

Preferred active compounds are selected from the group consisting of cisplatin, doxorubicin, and VP-16.

Other active compounds, which can be used for the treatment of tumor cells and self-reactive cells of the immune system overexpressing Smac include death receptor ligands, such as tumor necrosis factor α (TNF-α), tumor necrosis factor β (TNF-β, lymphotoxin-α), LT-β (lymphotoxin-β), TRAIL (Apo2L), CD95 (Fas, APO-1) ligand, TRAMP (DR3, Apo-3) ligand, DR4 ligand, DR6 ligand as well as fragments and derivatives of any of said ligands. Preferably, the death receptor ligand is selected from the group consisting of TNF-α, a fragment or derivative thereof, and TRAIL, a fragment and derivative thereof.

For the treatment of tumor cells overexpressing Smac there can also be used agonistic antibodies to death receptors such as anti-CD95 antibody, anti-TRAIL-R1 (DR4) antibody, anti-TRAIL-R2 (DR5) antibody, anti-DR6 antibody, anti TNF-R1 antibody and anti-TRAMP (DR3) antibody as well as fragments and derivatives of any of said antibodies. Preferably, the agonistic antibodies are selected from the group consisting of anti-TRAIL-R1 antibody, anti-TRAIL-R2 antibody, anti TNF-R1 antibody and fragments and derivatives of any of said antibodies.

The term derivative or fragment of the Smac gene refers to DNA sequences in which one or more nucleotides of the coding sequence of 1358 nucleotides, as disclosed in GenBank number AF262240, can be substituted by one or more nucleotides different from the original one(s), or Smac DNA sequences the nucleotide sequence of which is either extended, shortened, or both, on either the 5'-, or the 3'- or both ends, provided that the function of the encoded Smac protein remains unaffected.

A preferred fragment of the Smac gene in the present invention to be overexpressed in tumor cells include the Smac cDNA lacking the nucleotides 20-184 of the disclosed coding sequence, which codes for the so-called mitochondrial targeting sequence (aminoacids 1-55 of the corresponding Smac protein), thus enabling the overexpression of Smac directly in the cytosol, which is the preferred site of Smac action.

By the administration of an active compound combined with the overexpression of Smac in the cells to be treated, as described beforehand, neuroblastoma and glioblastoma and related types of cancer, like colon carcinoma, hepatocelluar carcinoma or small cell and non-small cell lung carcinoma, can be treated successfully. Thus, a further object of the present invention are kits comprising at least one active compound, as described above, and expression plasmids carrying the full length Smac gene, as disclosed in GenBank number AF262240, or a derivative or fragment thereof. The said kits can be used as a medicament for the treatment of neuroblastoma, glioblastoma and related cancers.

### EXAMPLES

**Overexpression of Smac sensitizes for death receptor or drug-induced apoptosis.** A full length Smac construct was used to transfect SHEP neuroblastoma cells, which exhibit intermediate sensitivity to various pro-apoptotic stimuli. Representative experiments performed with clone #28 which overexpressed high levels of Smac are subsequently done. Overexpression of Smac potentiated TRAIL-induced apoptosis in a dose- and time-dependent manner compared to vector control cells and also markedly increased apoptosis induced by anti-CD95 antibody or cytotoxic drugs. Because overexpression of Smac enhanced both death receptor and drug-induced apoptosis, Smac acts at a common point where these two pathways converge, e.g. at the level of postmitochondrial activation of caspases.

**Smac sensitizes for apoptosis by antagonizing XIAP**. It was investigated whether the apoptosis promoting effect of Smac was mediated by antagonizing XIAP, a prominent caspase inhibitor. Treatment with TRAIL resulted in enhanced release of Smac from mitochondria into the cytosol in cells transfected with Smac compared to vector control cells Immunoprecipitation of Flag-tagged Smac showed binding of Smac to XIAP upon treatment with TRAIL. Also, immunoprecipitation of endogenous XIAP revealed enhanced binding of Smac to XIAP in Smac transfected cells upon TRAIL treatment compared to vector control cells resulting in complete dissociation of XIAP from caspase-9. Furthermore, overexpression of Smac enhanced activation of caspase-8, -9, -3, cleavage of the caspase substrates PARP and DFF45 and cleavage of Bid and XIAP upon treatment with TRAIL or doxorubicin. These findings indicate that overexpression of Smac promoted apoptosis through antagonizing the inhibition of XIAP of both distal and proximal events in the caspase cascade.

**Cytosolic Smac bypasses the Bcl-2 inhibition.** Since Bcl-2 may prevent Smac release from mitochondria, Smac function was analyzed in SHEP neuroblastoma cells transfected with Bcl-2. Overexpression of Bcl-2 prevented the release of Smac and cytochrome c from mitochondria upon TRAIL treatment. Also, Bcl-2 inhibited activation of caspase-3 into active fragments and cleavage of the caspase-3 substrates PARP and DFF45. Interestingly however, Bcl-2 reduced, but did not prevent the initial cleavage of caspase-3 into the p24 intermediate fragment or cleavage of caspase-8 consistent with a block at the postmitochondrial level, e.g. by XIAP. It was investigated whether cytosolic Smac without the mitochondrial targeting sequence can bypass the Bcl-2 block. Ectopic expression of GFP-tagged Smac in the cytosol was controlled by fluorescence microscopy (data not shown). Importantly, ectopic expression of cytosolic Smac sensitized SHEP neuroblastoma cells overexpressing Bcl-2 for apoptosis induction. Also, cytosolic Smac further enhanced treatment-induced apoptosis in SHEP vector control cells, consistent with high XIAP expression in these cells. Expression of cytosolic Smac per se showed no cytotoxic effect indicating that the release from IAP inhibition by Smac only becomes relevant upon apoptosis induction. The studies were further extended to different cell lines with Bcl-2 overexpression. Ectopic expression of cytosolic Smac sensitized Bcl-2 transfected glioblastoma (U87MG/Bcl-2, LN18/Bcl-2, LN229/Bcl-2) and breast carcinoma (MCF7/Bcl-2) cells for treatment with TRAIL, anti-CD95 antibody or doxorubicin. Thus, cytosolic Smac may bypass Bcl-2 inhibition in several cell types and in response to different pro-apoptotic stimuli.

**Smac peptides sensitize resistant tumor cells for death receptor or drug-induced apoptosis.** The N-terminal 4 residues of Smac that are essential for inactivation of XIAP and thus for apoptosis induction, together with the 3 following residues, were linked to the protein transduction domain of the TAT protein to facilitate intracellular delivery (Smac peptide / PTD). Cellular uptake of Smac peptides was controlled by flow cytometry and fluorescence microscopy. Smac peptides markedly enhanced TRAIL-induced apoptosis and also sensitized for treatment with anti-CD95 antibody or cytotoxic drugs. Furthermore, Smac peptides sensitized several resistant cell lines with defects in apoptosis signaling for treatment with TRAIL or doxorubicin, including neuroblastoma cells with Bcl-2 overexpression (SHEP/Bcl-2), neuroblastoma cells with absent caspase-8 expression (SH-SY5Y), melanoma cells with impaired Apaf-1 expression (Mel-HO) or pancreatic carcinoma cells with defective Ras/PI3 Kinase/Akt signaling (Pant-1).

To exclude that the observations were restricted to cell lines maintained in long-term culture, primary tumor cells derived from a malignant pleural effusion of a patient with neuroblastoma at tumor relapse with refractory disease were examined. Importantly, Smac peptides sensitized these patient's derived resistant neuroblastoma cells with high levels of XIAP and Bcl-2, for apoptosis induced ex *vivo* by TRAIL or anticancer drugs.

**Smac peptides enhance the antitumor effect of TRAIL in glioblastoma *in vivo* and induce eradication of tumors.** The effect of Smac was examined in a glioblastoma tumor model *in vivo*. Glioma cells were implanted into the right striatum of athymic mice and Smac peptides and /or TRAIL were locally administered at day 7 and day 9 after tumor inoculation. Importantly, Smac peptides significantly sensitized glioblastoma cells for TRAIL-induced apoptosis, while treatment with Smac peptides alone showed no antitumor effect. Complete eradication of preestablished glioblastoma tumors was only found in mice treated with the combination of Smac peptides and TRAIL in 33% (2 of 6) or 50% (3 of 6) of tumors. Combined administration of Smac peptides and TRAIL showed no acute or delayed neurotoxicity as assessed by a compound neurological score, whereas 2 of 6 mice treated with TRAIL alone developed neurological deficits indicating that the combination of Smac peptides and TRAIL may also improve neurological outcome.

### MATERIALS AND METHODS

*Cell culture.* Neuroblastoma (SHEP, SH-SY5Y), glioblastoma (U87MG, LN18, LN229), Panc-1 pancreatic carcinoma or MCF-7 breast carcinoma were maintained in RPMI 1640 medium (Life Technologies, Inc., Eggenstein, Germany) as previously described. 0.5 x 10⁵ cells/ml were cultured in 24-well-plates for determination of apoptosis or in 75 cm² flasks (Falcon, Heidelberg, Germany) for protein isolation.

*Determination of apoptosis*. Cells were incubated with recombinant human TRAIL (PeproTech Inc., Rocky Hill, NJ), cisplatin (Sigma, Deisenhofen, Germany), doxorubicin (Amersham Pharmacia, Freiburg, Germany) VP-16 (Bristol Myers, Erlangen, Germany) or anti-CD95 (APO1) monoclonal antibody. Smac peptides corresponding to aa 56-62 were linked to the protein transduction domain of Tat protein (Interactiva GmbH, Ulm, Germany). For assessment of cellular uptake, FITC-labelled peptides were used. Quantification of DNA fragmentation was performed by fluorescence-activated cell-sorting (FACS) analysis of propidium iodide stained nuclei as previously described.

*Western blot analysis and immunoprecipitation*. Western blot analysis and immunoprecipitation were performed as previously described using mouse anti-caspase-8 monoclonal antibody C15 (1:10 dilution of hybridoma supernatant), mouse anti-caspase-3 monoclonal antibody (1:1000, Transduction Laboratories, Lexington, KY), rabbit anti-caspase-9 polyclonal antibody (1:1000, PharMingen, San Diego, CA), mouse anti-XIAP monoclonal antibody (1:1000, H62120, Transduction Laboratories), mouse anti-DFF45 monoclonal antibody (1:1000, Transduction Laboratories), rabbit anti-AIF polyclonal antibody (1:5000, kindly provided by G. Kroemer), rabbit anti-Smac polyclonal antibody (1:5000, kindly provided by X. Wang), mouse anti-COX4 monoclonal antibody (1:1000, Clontech Laboratories, Inc., Palo Alto, CA), mouse anti-Flag monoclonal antibody (1:1000, Sigma) or mouse anti-β-actin monoclonal antibody (1:5000, Sigma) followed by goat anti-mouse IgG or goat anti-rabbit IgG (1:5000, Santa Cruz Biotechnology, Santa Cruz, CA). Enhanced chemiluminescence (ECL, Amersham Pharmacia) was used for detection. Expression of β-actin was used to control for equal gel loading.

*Transfection experiments*. SHEP neuroblastoma cells were transfected with expression plasmid pcDNA3.1 vector containing full length Smac cDNA or empty vector using lipofectamine transfection reagent (Life Technologies, Inc.) and cultured in 0.5 mg/ml G418 (Life Technologies, Inc.). Transient transfections with pEGFPC1 vector containing GFP-tagged Smac without the mitochondrial targeting sequence (aa 1-55) ²⁶ were performed using gene porter transfection reagent.

*Preparation of mitochondria or cytosolic extracts.* Preparation of mitochondria or cytosolic extracts was performed using the ApoAlert cell fractionation kit (Clontech Laboratories) according to the manufacturer's instructions.

*Animal studies.* 5 x 10⁴ U87MG human glioblastoma cells were stereotactically implanted into the right striatum of athymic mice (CD nu/nu, Charles River, Sulzfeld, Germany). At day 7 or at day 7 and day 9, mice were locally treated with Apo2L/TRAIL (2 µg/4 µl buffer) and/or Smac (1 mg/4 µl buffer) or buffer only. Tumor cell volumes were measured at day 21 or 35 after tumor cell implantation as previously described. Neurological symptoms (alertness, behaviour, weight loss, focal neurological deficits) were evaluated daily and a compound score of all categories was formed (++: severe deficits, +: deficits, -: no relevant deficits). Statistical significance was assessed using ANOVA.

## Claims

1. A Smac protein / carrier entity comprising
(i) a Smac protein, as disclosed by the GenBank accession number AAF87716, or a derivative or fragment thereof,
(ii) a carrier
and wherein the Smac protein, fragment or derivative thereof and the carrier are linked together enabling the penetration of the Smac/carrier entity through the cell membrane into the cell.

2. The entity according to claim 1, wherein the fragment or derivative of Smac is a peptide comprising the aminoacid sequence 56 to 70.

3. The entity according to claim 1 or 2, wherein the fragment or derivative of Smac is a peptide comprising aminoacids 56 to 62 of Smac.

4. The entity according to any of claims 1 to 3, wherein the fragment or derivative of Smac comprises the aminoacids 56 to 59 of Smac.

5. The entity according to any of claims 1 to 4, wherein said carrier is a protein, a fragment or derivative thereof.

6. The entity according to any of claims 1 to 5, wherein said carrier is selected from the group consisting of TAT, influenza virus hemagglutinin, the VP22 protein from herpes simplex virus, Antennapedia, fibroblast growth factor, Galparan (transportan), poly-arginine, and Pep-1, and fragments and derivatives thereof, and lipids and cationic lipids.

7. The entity according to any of claims 1 to 6, wherein said protein is the TAT protein or a fragment or derivative thereof, as disclosed by GenBank accession number CAA45921.

8. The entity according to any of claims 1 to 7, wherein the fragment or derivative of the TAT protein comprises the aminoacids 37 to 72 of TAT.

9. The entity according to any of claims 1 to 8, wherein said carrier is the protein transduction domain of TAT comprising the aminoacids 47 to 57 of TAT.

10. The entity according to any of claims 1 to 9, optionally in combination with at least one active apoptosis-inducing or proliferation-inhibiting compound for use as pharmaceutical.

11. The entity for use as pharmaceutical according to claim 10, wherein the active compound is a cytostatic compound.

12. The entity for use as a pharmaceutical according to claims 10 or 11, wherein the cytostatic compound is selected from the group consisting of antimetabolites, preferably cytarabine, fludarabine, 5-fluoro-2'-deoxyuiridine, gemcitabine, hydroxyurea or methotrexate; DNA-fragmenting agents, preferably bleomycin, DNA-crosslinking agents, preferably chlorambucil, cisplatin, cyclophosphamide or nitrogen mustard; intercalating agents preferably adriamycin (doxorubicin) or mitoxantrone; protein synthesis inhibitors, preferably L-asparaginase, cycloheximide, puromycin or diphteria toxin; topoisomerase I poisons, preferably camptothecin or topotecan; topoisomerase II poisons, preferably etoposide (VP-16) or teniposide; microtubule-directed agents, preferably colcemid, colchicine, paclitaxel, vinblastine or vincristine; kinase inhibitors preferably flavopiridol, staurosporin, STI571 (CPG 57148B) or UCN-01 (7-hydroxystaurosporine); miscellaneous investigational agents, preferably PS-341, phenylbutyrate, ET-18-OCH₃, or farnesyl transferase inhibitors (L-739749, L-744832); polyphenols preferably quercetin, resveratrol, piceatannol, epigallocatechine gallate, theaflavins, flavanols, procyanidins, betulinic acid; hormones preferably glucocorticoids or fenretinide; hormone antagonists, preferably tamoxifen, finasteride or LHRH antagonists; plant-derived cytostatics (from Viscum and derivatives); alcaloids preferably vindesine; podophyllotoxins preferably vinorelbin; alkylants preferably nimustrine, carmustrine, lomustine, estramustrine, melphalam, ifosfamide, trofosfamide, bendamustine, dacarbazine, busulfane, procarbazine, treosulfane, tremozolamide, thiotepa; cytotoxic antibiotics preferably aclarubicine, daunorubicine, epirubicine, idarubicine, mitomycine, dactinomycine; antimetabolites like folic acid analogs preferably methotrexate, purine analogs preferably cladribin, mercaptopurin, tioguanine and pyrimidine analogs preferably cytarabine, fluorouracil, docetaxel; other antineoplastic, platinum compounds preferably thioplatin, carboplatin, oxaliplatin; amsacrine, irinotecane, interferon-α, tretinoine, hydroxycarbamide, miltefosine, pentostatine, aldesleukine; antineoplastic compounds derived from organs, e.g. monoclonal antibodies preferably trastuzumab, rituximab, or derived from enyzmes preferably pegaspargase; endocrine effecting antineoplastic compounds belonging to hormones, e.g. estrogens preferably polyestradiol, fosfestriol, ethinylestradiol, gestagens preferably medroxyprogesterone, gestonoroncaproat, megestrol, norethisterone, lynestrenol, hypothalamus hormones preferably triptoreline, leuproreline, busereline, gosereline, other hormones preferably testolactone, testosterone; endocrine effecting antineoplastic compounds belonging to hormone antagonists, e.g. antiestrogens preferably toremifen; antiandrogens preferably flutamide, bicalutamide, cyproterane; endocrine effecting antineoplastic compounds belonging to enzyme inhibitors preferably anastrol, exemestane, letrozol, formestane, aminoglutethimide, all of which can be occasionally administered together with so-called protectives preferably calciumfolinat, amifostin, lenograstin, molgromostin, filgrastin, mesna or so-called additives preferably retinolpalmitate, thymus D9, amilomer.

13. The entity for use as a pharmaceutical according to any of claims 10 to 12, wherein the cytostatic compound is selected is from the group consisting of doxorubicin, cisplatin and etoposide (VP-16).

14. The entity for use as a pharmaceutical according to claim 10, wherein the active compound is a death receptor ligand, derivative or fragment thereof.

15. The entity for use as a pharmaceutical according to claim 14, wherein the death receptor ligand is selected from the group consisting of tumor necrosis factor α (TNF-α), tumor necrosis factor β (TNF-β, lymphotoxin-α), LT-β (lymphotoxin-β), TRAIL (Apo2L), CD95 (Fas, APO-1) ligand, TRAMP (DR3, Apo-3) ligand, DR4 ligand, DR6 ligand as well as fragments and derivatives of any of said ligands.

16. The entity for use as a pharmaceutical according to claims 14 or 15, wherein the death receptor ligand is TRAIL.

17. The entity for use as a pharmaceutical according to claim 10, wherein the active compound is an antibody against a death receptor, a derivative or fragment thereof.

18. The entity for use as a pharmaceutical according to claim 17, wherein the antibody against the death receptor ligand is selected from the group consisting of anti-CD95 antibody, anti-TRAIL-R1 (DR4) antibody, anti-TRAIL-R2 (DR5) antibody, anti-DR6 antibody, anti TNF-R1 antibody and anti-TRAMP (DR3) antibody as well as fragments and derivatives of any of said antibodies.

19. The entity for use as a pharmaceutical according to claims 17 or 18, wherein the antibody against the death receptor is the anti-CD95 antibody.

20. The use of Smac/carrier entity according to any of claims 1 to 9, optionally in combination with at least one active apoptosis-inducing compound for the manufacture of a medicament for the treatment of cancer.

21. The use according to claim 20, wherein the cancer to be treated is selected from a group consisting of neuroblastoma, rectum carcinoma, colon carcinoma, familiary adenomatous polyposis carcinoma, hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors preferably glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellualar carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma.

22. The use according to claim 20 or 21, wherein the cancer to be treated is selected from the group consisting of neuroblastoma, glioblastoma, breast carcinoma, melanoma, prostate cancer and pancreatic carcinoma.

23. A medicament for the treatment of cancer, comprising a Smac/carrier entity as claimed in any of the claims 1 to 9 and a pharmaceutically acceptable carrier.

24. The use of Smac/carrier entity according to any of claims 1 to 9, optionally in combination with at least one active apoptosis-inducing compound for the manufacture of a medicament for the treatment of autoimmune diseases.

25. The use according to claim 24, wherein the autoimmune disease to be treated is selected from a group consisting of collagen diseases particularly rheumatoid arthritis, Lupus erythematodes disseminatus, Sharp syndrome, CREST syndrome (calcinosis, Raynaud syndrome, esophageal dysmotility, teleangiectasia), dermatomyositis, vasculitis (Morbus Wegener) and Sjögren syndrome, renal diseases particularly Goodpasture syndrome, rapidly-progressing glomerulonephritis and membrane-proliferative glomerulonephritis type II, endocrine diseases particularly type-I diabetes, autoimmune polyendocrinopathy-candidiasis-ectodermal dystrophy (APECED), autoimmune parathyreoidism, pernicious anemia, gonad insufficiency, idiopathic Morbus Addison, hyperthyreosis, Hashimoto thyreoiditis and primary myxedemia, skin diseases particularly Pemphigus vulgaris, bullous pemphigoid, Herpes gestationis, Epidermolysis bullosa and Erythema multiforme major, liver diseases particularly primary biliary cirrhosis, autoimmune cholangitis, autoimmune hepatitis type-1, autoimmune hepatitis type-2, primary sclerosing cholangitis, neuronal diseases particularly multiple sclerosis, Myastenia gravis, myasthenic Lambert-Eaton syndrome, acquired neuromyotony, Guillain-Barré syndrome (Müller-Fischer syndrome), Stiff-man syndrome, cerebellar degeneration, ataxia, opsoklonus, sensoric neuropathy and achalasia, blood diseases particularly autoimmune hemolytic anemia, idiopathic thrombocytopenic purpura (Morbus Werlhof), infectious diseases particularly AIDS, Malaria and Chagas disease.

26. A medicament for the treatment of autoimmune diseases, comprising a Smac/carrier entity as claimed in any of the claims 1 to 9 and a pharmaceutically acceptable carrier.

27. The use of an expression plamid carrying the full length Smac gene, as disclosed by GenBank accession number AF262240, or a derivative or a fragment thereof, in combination with an active compound for the manufacture of a medicament for the treatment of neuroblastoma, glioblastoma, prostate carcinoma, colon carcinoma, small cell and non-small cell lung carcinoma.

28. The use according to claim 27, wherein the full length Smac gene as disclosed is substituted by a Smac DNA fragment lacking the nucleotides 20 to 184 of the disclosed coding sequence.

29. The use according to claim 27 or 28, wherein the active compound is selected from the group of cytostatic compounds consisting of cisplatin, doxorubicin, and VP-16.

30. The use according to claim 27 or 28, wherein the active compound is selected from the group of death receptor ligands consisting of tumor necrosis factor α (TNF-α), tumor necrosis factor β (TNF-β, lymphotoxin-α), LT-β (lymphotoxin-β), TRAIL (Apo2L), CD95 (Fas, APO-1) ligand, TRAMP (DR3, Apo-3) ligand, DR4 ligand, DR6 ligand as well as fragments and derivatives of any of said ligands.

31. The use according to claim 30, wherein the death receptor ligand is TRAIL.

32. The use according to claim 27 or 28, wherein the active compound is an antibody against a death repector.

33. The use according to claim 32, wherein the antibody against a death receptor is the anti-CD95 antibody.

34. A kit, comprising at least one active compound, as described above, and expression plasmids carrying the full length Smac gene, as disclosed in GenBank number AF262240, or a derivative or fragment thereof.

35. The use of the kit according to claim 34 for the manufacture of a medicament for the treatment of neuroblastoma, glioblastoma, prostate cancer, colon cancer, hepatocellular carcinoma, small cell lung cancer and non-small cell lung cancer and related cancers.
